(19) Europäisches Patentamt
European Patent Office
Office européen des brevets

(11) Publication number: **0 022 148**
**B1**

(12) # EUROPEAN PATENT SPECIFICATION

(45) Date of publication of patent specification: **07.11.84**

(51) Int. Cl.$^3$: **C 08 G 73/00**, C 08 L 79/00, A 01 N 59/12

(21) Application number: **80101909.2**

(22) Date of filing: **10.04.80**

(54) **Complexes of polyoxazolines or polyoxazines with polyhalide anions, their preparation, method and sanitizing agents comprising them.**

(30) Priority: **16.04.79 US 30396**

(43) Date of publication of application:
**14.01.81 Bulletin 81/02**

(45) Publication of the grant of the patent:
**07.11.84 Bulletin 84/45**

(84) Designated Contracting States:
**DE FR GB NL**

(56) References cited:
**US-A-4 144 211**

**The file contains technical information submitted after the application was filed and not included in this specification**

(73) Proprietor: **THE DOW CHEMICAL COMPANY**
**Dow Center 2030 Abbott Road Post Office Box 1967**
**Midland Michigan 48640 (US)**

(72) Inventor: **Ginter, Sally Pettengill**
**1120 W.Curtis Road**
**Sanford Michigan (US)**
Inventor: **Hamlin, Percy Jay**
**1418 E.Haley**
**Midland Michigan (US)**

(74) Representative: **Casalonga, Alain et al**
**BUREAU D.A. CASALONGA OFFICE JOSSE & PETIT Baaderstrasse 12-14**
**D-8000 München 5 (DE)**

**EP 0 022 148 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to halophors. More particularly, the invention relates to a novel class of complexes of 2-oxazoline or 2-oxazine polymers and polyhalide anions, to methods of making these complexes and to the use of these complexes as sanitizing agents.

Of the many known halophors, probably the most common are those prepared from iodine ($I_2$) and polyvinylpyrrolidone. These materials are generally complexes of indefinite composition and are generally soluble in water. When solubilized, these complexes slowly liberate free iodine which is the active ingredient. These complexes can contain up to about 25 weight percent iodine although not all is available (titratable with $Na_2S_2O_3$) due to some being organically bound to the polymer.

Halophors or complexes of polyoxazolines or polyoxazines and halogens, interhalogens or pseudohalogens, are described in U.S. Patent 4,144,211 by Chamberlin and Bangs. These complexes represent a class of halophors distinct from the complexes of polyvinylpyrrolidone and have demonstrated utility as sanitizing agents. However, like complexes of polyvinylpyrrolidone, the total weight percent of iodine that these materials can complex is not entirely satisfactory.

This invention provides water-soluble complexes useful as sanitizing agents which comprise (a) a poly-2-oxazoline or poly-2-oxazine or the corresponding partially deacylated polymer and (b) $IBrCl^-$ or a polyhalide anion of the formula $(XY_{2n})^-$ where X and Y are individually chloride, bromide or iodide, but not both chloride, and n is 1, 2 or 3, and at least one independently supplied cation of hydrogen or an alkali or alkaline earth metal. The preferred cations are potassium, sodium and hydrogen, with hydrogen being the most preferred. Other alkali or alkaline earth metal cations, such as lithium, magnesium, and calcium, may be employed if desired.

These materials are significantly more stable than prior art complexes of either poly-2-oxazoline or poly-2-oxazine and halogen, interhalogen or pseudo-halogen or complexes of polyvinylpyrrolidone and polyhalides. Moreover, not only can the complexes of this invention be prepared with a significantly greater $I_2$:polymer ratio than can analagous polyvinylpyrrolidone complexes at equivalent percent solid levels, but the instant complexes have a significantly lower viscosity than do analagous polyvinylpyrrolidone complexes at equivalent iodine loadings. This latter distinction is particularly significant because it permits the preparation of concentrated, aqueous, polymer-polyhalide complex solutions previously not obtainable with prior art complexes, notably polyvinylpyrrolidone-polyhalide complexes.

The poly-2-oxazolines and poly-2-oxazines are known compounds consisting essentially of m units (I and/or II), randomly joined, and are readily prepared by the ring-opening polymerization of substituted oxazolines or oxazines (III), followed optionally by hydrolysis.

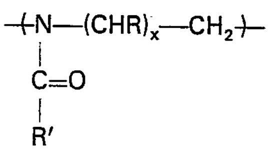

(I)

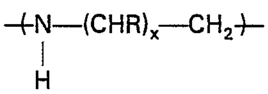

(II)

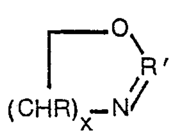

(III)

The substituents and subscript are later defined. The ring-opening polymerization is generally conducted in the presence of a cationic polymerization catalyst at a reaction temperature of 0° to 200°C. Typical catalysts include strong mineral acids, organic sulfonic acids and their esters, acidic salts such as ammonium sulfate, Lewis acids such as aluminum trichloride, stannous tetrachloride, boron trifluoride, and organic diazoniumfluoroborates dialkyl sulfates, and other like catalysts. This ring-opening polymerization is further described by Tomalia et al., *Journal of Polymer Science*, 4, 2253 (1966); Bassiri et al., *Polymer Letters*, 5, 871 (1967); and Seeliger, German Patent 1,206,585.

The polymers thereby obtained are linear poly-2-oxazolines or poly-2-oxazines having a molecular structure consisting essentially of m repeating units (I). The polymers are easily deacylated by acid or base hydrolysis but since hydrolysis (deacylation) is generally best controlled under acidic conditions, acid hydrolysis is preferred. The partially deacylated poly-2-oxazolines or poly-2-oxazines thus have a

molecular structure consisting essentially of m randomly joined units (I) and (II), illustratively depicted as

$$\begin{array}{ll} -\!\!(\text{N}-(\text{CHR})_x-\text{CH}_2)_h & (\text{N}-(\text{CHR})_x-\text{CH}_2)_{m-h} \\ \;\;| & \;\;| \\ \;\;\text{C}-\text{O} & \;\;\text{H} \\ \;\;| & \\ \;\;\text{R}' & \end{array} \qquad \text{(IV)}$$

wherein: m is the total number of units; h is the number of acylated units; and m-h is the number of deacylated units.

"Poly-2-oxazolines" and "poly-2-oxazines" here include both the fully acylated and partially deacylated polymers. Partially deacylated polymers have at least one acyl group (R'C=O) per polymer chain, i.e. h is at least one. Preferably, the polymers are no more than 50 percent deacylated (h is at least 50 percent of m) and most preferably no more than 25 percent deacylated (h is at least 75 percent of m). Fully acylated or nondeacylated polymers (h is 100 percent of m) are most preferred.

In the above formulae, R is hydrogen of $C_1$ to $C_3$ alkyl, R' is a $C_1$ to $C_4$ alkyl, and x is 1 (an oxazoline) or 2 (an oxazine). Exemplary R substituents include hydrogen, methyl, ethyl and propyl and exemplary R' substituents include methyl, ethyl, propyl.

Any member of the known classes of poly-2-oxazolines and poly-2-oxazines can be used. Examples of suitable monomers from which the polymers (the term as here used includes copolymers) can be prepared include 2-H-2-oxazoline, 2-methyl-2-oxazoline, 2-ethyl-2-oxazoline, 2-propyl-2-oxazoline, 2-ethyl-4-methyl-2-oxazoline, 2-ethyl-5-methyl-2-oxazoline, 2-ethyl-5-dimethyl-2-oxazoline, 2-H-2-oxazine, 2-methyl-2-oxazine, 2-ethyl-2-oxazine and combinations thereof. Poly-2-oxazolines (x is 1) are preferred to the poly-2-oxazines (x is 2) and poly-2-oxazolines wherein R is hydrogen are more preferred. Homopolymers prepared from either 2-ethyl-2-oxazoline (V) or 2-propyl-2-oxazoline (VI) are especially preferred.

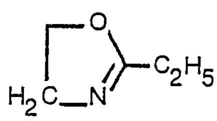

(V)

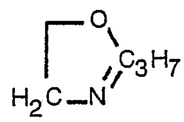

(VI)

Polymers of particular interest useful for preparing the complexes of this invention are functionalized polymers, i.e. polymers prepared from a hydrophobeinitiated, ring-opening polymerization of 2-oxazoline and/or 2-oxazine monomers. These functionalized polymers are characterized by the presence of a hydrophobe radical attached to either or both (typically just one) terminal mer units of the polymer and can be illustratively depicted as

$$\begin{array}{lll} \text{hydrophobe} & -\!\!(\text{N}\;-\!\!(\text{CHR})_x-\text{CH}_2)_n- & (\text{N}-\!\!(\text{CHR})_x-\text{CH}_2)_{m-h} \\ & \;\;| & \;\;| \\ & \;\;\text{C}=\text{O} & \;\;\text{H} \\ & \;\;| & \\ & \;\;\text{R}' & \end{array} \qquad \text{(VII)}$$

where the substituents and subscripts are as previously defined. Any organic compound that can initiate a ring-opening polymerization of 2-oxazoline and/or 2-oxazine monomer and which imparts detectable hydrophobic character to the resulting polymer can become the hydrophobe of the functionalized polymer. Typical examples of such compounds are the sulfonyl halides of long chain hydrocarbons, such as $C_8$ to $C_{40}$ alkyls, aralkyls, esters of inorganic acids, $C_8$ to $C_{40}$ alkyl halides and diphenyl ether sulfonyl halides. Polyisobutylene and dodecylbenzene sulfonyl chloride are illustrative. The functionalized polymers exhibit desirable surfactant properties and when complexed with polyhalide anions, provide a most unique sanitizing agent.

The nonfunctionalized polymers used in this invention typically have a weight average molecular weight of at least 10,000 as determined by gel chromatography. More typically, these compounds have an average minimum molecular weight of 20,000 and preferably of 100,000. Practical considerations, such as preparation and handling are the only limitations upon the average maximum molecular weight of these polymers, although convenience prefers a maximum of 800,000 and, more preferably, of 500,000.

The functionalized polymers typically consist of less than 20 mer units, and more typically less than 10 mer units, and thus have a typical maximum molecular weight less than 3000, and more typically less than 1000. Typically, these polymers comprise at least two mer units and more typically at least 4 mer units and thus have a typical minimum molecular weight of at least 250, and more typically of at least 500. The exact molecular weight of these polymers, especially the polymers of few mer units, is significantly dependent upon the molecular weight of the hydrophobe. Also, the molecular

weight of these polymers, and the nonfunctionalized polymers as well, is related to the definitions of R, R′ and X. Thus where R and R′ are the larger members (based in molecular weight) of their respective classes, the 3000 typical maximum molecular weight for functionalized polymers may be exceeded.

The polyhalide anions used are $IBrCl^-$ or one of the formula $(XY_{2n})^-$ where the substituents are as previously defined. Typical polyhalide anions include $Br_3^-$, $Br_5^-$, $Br_7^-$, $I_3^-$, $I_5^-$, $I_7^-$, $BrI_2^-$, $ClI_2^-$, $IBr_2^-$, $ClBr_2^-$, $BrCl_2^-$, $ClBr_4^-$, $ICl_2^-$, etc. Polychloride anions are unstable at ambient temperature unless maintained in an atmosphere of chlorine. Dissociation of fluorine containing polyhalides is also too high for practical use at ambient temperature. The polyhalide anions of either or both bromine and iodine are preferred, with the polyhalide anions of iodine especially preferred. The most preferred anions are those where n is 1.

It is a characteristic of this invention that the cations are independently supplied, i.e. they are not taken from the polymer. The most typical source of the cations is a halide salt which is added to convert a halogen into a polyhalide. For example, KI is added to $I_2$ to form $KI_3$. The potassium cation comes from a source external to the polymer. The cation may be incorporated in the composition in a number of ways as is readily apparent to those skilled in the art.

The cation will be present in an amount of at least 10 percent of the stoichiometric equivalent of the polyhalide anion and is preferably present in an amount of at least 90 percent of the stoichiometric equivalent. Most preferably, the cation will be present in an amount equal to the stoichiometric equivalent of the polyhalide anion, although a stoichiometric excess may be employed if desired.

The complexes of this invention are readily prepared at temperatures of 0° to 100°C but are preferably prepared at ambient temperature in either an aqueous or anhydrous state. Aqueous preparation is preferred and may be exemplified by contacting an aqueous solution or slurry or a poly-2-oxazoline or poly-2-oxazine with an aqueous solution of iodine and an alkali metal iodide or HI. The resulting aqueous complex can then be recovered as a solid by simply subjecting the aqueous complex to drying.

A typical anhydrous preparation involves, for example, mixing an aqueous solution of a poly-2-oxazoline or poly-2-oxazine with an alkali metal iodide, drying the resulting mixture and subsequently grinding it to a relatively fine powder. The dried, ground polymer-iodide mixture is then blended with solid iodine at a suitable ratio, the blending accomplished by any convenient means, such as milling, rolling. The known procedures for preparing polyvinylpyrrolidone-polyiodide solid complexes may be used for preparing the polymer-polyhalide solid complexes of this invention.

The composition of the complexes of this invention can vary considerably. Typically, the polymer:polyhalide anion weight ratio ranges from 99:1 to 35:65 and, preferably from 85:15 to 50:50.

Polyhalide anions are formed from the combination of a halide anion, such as chloride, bromide or iodide, with either a halogen or an interhalogen, such as chlorine, bromine, iodine, bromine chloride (BrCl), iodine chloride (ICl), iodine bromide (IBr), iodine trichloride ($ICl_3$), etc. As such, the complexes of this invention contain some amounts of all three entities, i.e. polyhalide anion, halogen, and halide anion. The polyhalide anion is present in the complex when the halogen or interhalogen:halide ratio is at least 1:0.1, and preferably at least 1:0.4. The typical maximum ratio is 1:2 and generally 1:1. Such amounts of halogen or interhalogen and halide maintain the presence of polyhalide anion in the complex.

Complexes of polyvinylpyrrolidone and polyhalide anion contain at least minor amounts of bound halide, i.e. halide chemically bound to the polymer. The bound halide in these polymers is lost halogen, i.e. halogen not available for sanitizing purposes, and is thought to be a result of uptake of the halide by the technical unsaturation of the polvyinylpyrrolidone. Since the polymers of 2-oxazoline and/or 2-oxazine have no such unsaturation, the amount of bound halide present in the instant complexes is essentially nil.

The solid complexes are usually colored solids which are soluble in polar solvents, such as water, methanol, tetrahydrofuran, isopropanol and essentially insoluble or only slightly soluble in relatively nonpolar solvents, such as methylene chloride, methyl cyanide, carbon tetrachloride. The solid complexes are essentially odorless and their color varies, depending principally upon the type of halogen or interhalogen present. For example, a complex of poly(2-ethyl-2-oxazoline)/polyiodide anion is brown/black to amber/brown (depending upon particle size) while the same polymer complex with polybromide is of an orange color. Like polymers complexed with $ICl_2^-$ or $BrCl_2^-$ are of a yellow color. This invention includes both the solid complexes and their various solutions. An aqueous solution of poly(2-ethyl-2-oxazoline)/polyiodide is of a reddish brown color while a solution of the same polymer complexed with polybromide, like the corresponding solid, is of an orange color. The solutions, like the solid complexes, are essentially odorless.

The complexes of this invention are used in the same manner as known halophor complexes. These complexes are useful as sanitizing agents due to their oxidative properties and thus find a multiplicity of uses in the medicinal and purification arts. Moreover, these complexes can be used in combination with other materials such as anionic and non-anionic detergents, or in combination with other halophors.

One of the advantages of this invention is the high, total weight percent halogen that can be complexed with poly-2-oxazoline or poly-2-oxazine. Prior art complexes do not provide the same

degree of total weight percent halogen complex per unit of polymer. As such, the complexes of the instant invention permit the use of less polymer (the inert component of these complexes) for binding essentially the same amount of halogen. Moreover, compared to the complexes of polyvinylpyrrolidone, the instant complexes are highly soluble in water, their aqueous solutions are thus much less viscous on an equivalent percent solids basis, and thus the preparation of aqueous concentrates high in halogen content are more easily prepared. Such concentrates are desirable from a commercial perspective because they are easier to ship and store than their solid counterparts, avoiding such problems as dusting, etc. Further, more halogen is bound in available form by the complexes of this invention than those of the prior art. Still further, the instant complexes demonstrate relatively long shelf stability, little if any odor, little if any irritation to skin, and little if any corrosive effects.

The following examples are illustrative of certain specific embodiments of this invention. Unless otherwise noted, all parts and percentages are by weight.

The terms and tests used are well-known in the art. The distribution coefficient (D.C.) is determined by adding 1.00 ml of a previously titrated test solution to 25 ml of heptane in a stoppered glass container. The container is placed in a bath maintained at 25°C$\pm$1° while it is agitated vigorously for one minute. The solution is then allowed to stand for a few minutes before sampling of the clear heptane layer by pipette. Iodine in heptane layer is determined at 520 nm, the absorption peak; the relationship between absorbance and iodine concentration in this solvent is linear throughout the range 1 to 25 mg per 100 ml. Using a Beckman model DV spectrophotometer, an absorption of 0.142 corresponded to 1.00 mg iodine extracted 25 ml heptane. The iodine remaining in the aqueous phase is determined by difference. The distribution coefficient is calculated by the following formula:

$$\text{D.C.} = \frac{\text{mg } I_2 \text{ remaining in aq. phase}}{\text{mg } I_2 \text{ in heptane}} \times \frac{\text{ml heptane}}{\text{ml aq. phase}}$$

Values so obtained are readily reproducible to within 10 percent and frequently to within 1 percent. Such terms as "total iodine (or halogen)", "Volhard/Iodine (or halogen)", etc. are defined in "Detergent/Iodine Systems", *Soap and Chemical Specialties,* (August, 1967) by Schmidt and Winicov. These terms are also widely used in the patent literature.

## Example 1
### Aqueous Solution Complex Preparation

A stock solution of iodine and potassium iodide in water was prepared by mixing 40 parts iodine, 26 parts potassium iodide and 34 parts water. 17.5 Part of this solution were then mixed with 82.5 parts of a 15.8 percent aqueous poly(2-ethyl-2-oxazoline) (PEOx) solution. The PEOx had a weight average molecular weight of about 100,000. The two solutions were mixed by stirring until a homogeneous solution of the two was formed (less than one hour). The resulting complex was determined to have a DC of 260 when 1 ml of the 1.75 percent (as iodine) aqueous solution was extracted into 25 ml of n-heptane at 25°C$\pm$1°C.

## Example 2

Aqueous complexes of PEOX/$I_3^-$ were prepared as in Example 1 where the PEOx:$I_2$ ratio was held at 90:10 and the halide varied. HI, KI and NaI were evaluated at an $I_2$:iodide weight ratio of 1:0.5. The resulting complexes were determined to have DC values of 364, 260 and 208 respectively, when 1 ml of a 1.75 percent (as iodine) aqueous solution was extracted into 25 ml of n-heptane at 25°C$\pm$1°C.

## Example 3 and Control A
### Anhydrous Complex Preparation

The solid complexes of both PEOx/iodine/iodide and polyvinylpyrrolidone (PVP)/iodine/iodide were prepared by the procedure outlined in U.S. Patent 3,898,326 issued to Cautor et al., August 5, 1975. The PEOx had a weight average molecular weight of about 100,000 and the PVP had a K value of 30 and a number average molecular weight of about 40,000. A solution of the respective polymers and sodium iodide (NaI) (2 parts polymer:1.3 parts NaI was dried and subsequently ground in a blender. The dried polymer was then sieved and the fraction that passed a 200 mesh screen (sieve opening 0.074 mm) was used for iodine complexing. The polymer-NaI composition was blended with iodine in ratios of 76.3:23.7, 69.2:30.8 and 61.7:38.3 by rolling on laboratory rolls for about 3 hours. After about 70 hours, the samples were subjected to a starch response time test (SRT). (A strip of moistened starch-iodide paper was held approximately 60 mm above the various complexes in a closed bottle. The time to develop any blue color on the paper was noted as the SRT).

The complexes were also compared in a chloroform test (CT). The samples (0.5 g) of each complex were mixed with chloroform (5 ml) and shaken for 30 seconds. The mixture was then

centrifuged and the color of the chloroform layer evaluated visually and by spectrophotometry at the iodine absorption peak (520 nm).

The results of this example and control are reported in Table I. Also there reported are the manufacturing specifications for each sample and the initial titratable or available iodine.

TABLE I

ANHYDROUS POLYMER-IODIDE-IODINE COMPLEXES

| Parts by Weight | | | | | | | | |
|---|---|---|---|---|---|---|---|---|
| PVP-Iodide | (Provides) (PVP) | ($I^-$) | Pulv'd Iodine | Manufacturing Ratio Polymer:$I^-$:$I_2$ | Initial Titratable* Iodine (%) | SRT | CT Abs. | CT Color** |
| 76.3 | (48) | (23.7) | 23.7 | 2/1/1 | 23.2 | 4 min | 0.145 | 2 |
| 69.2 | (43.9) | (21.4) | 30.8 | 1.4/0.7/1 | 29.7 | 1 min | 0.585 | 3 |
| 61.7 | (39.2) | (19.1) | 38.3 | 1/0.5/1 | 37.9 | 10 s | 1.95 | 4 |
| PEOx-Iodide | | | | | | | | |
| 76.3 | (48) | (23.7) | 23.7 | 2/1/1 | 24.6 | >20 min | 0.045 | 1 |
| 69.2 | (43.9) | (21.4) | 30.8 | 1.4/0.7/1 | 29.6 | 4 min | 0.11 | 2 |
| 61.7 | (39.2) | (19.1) | 38.3 | 1/0.5/1 | 37.8 | 45 s | 0.69 | 3 |

* With 0.1 N $Na_2S_2O_3$

**Color Code

1 — Slight Pale Wine-Red
2 — Pale Wine-Red
3 — Wine-Red
4 — Deep Wine-Red

The above data demonstrates several advantages of this invention. The SRT shows that less iodine escapes from the PEOx complexes than from the PVP complexes. This indicates greater shelf stability. The CT shows that the PEOx complexes extracted to a lesser degree than the PVP complexes at each iodine:iodide ratio as evidenced by less color imparted to the chloroform and consequently lower absorptions. Again, this indicates that the PEOx complexes are more stable than the PVP complexes.

Example 4

Aqueous PEOx-Bromine-Bromide Complex

Three solutions were prepared to demonstrate PEOx complexes with bromine and the effect of bromide (as potassium bromide) on the complex. Bromine was added to aqueous PEOx (weight average molecular weight of about 100,000) solutions (or aqueous PEOx and potassium bromide solutions when potassium bromide was included) and then placed on a mechanical shaker until dissolution occurred (generally after a few minutes). The solutions were then evaluated visually for bromine fumes. The results are reported in Table II.

In the solution where aqueous PEOx and bromine (no potassium bromide) was used, a portion (15 percent) of the bromine was converted to bromide (i.e., titratable with silver nitrate but not with sodium thiosulfate). The bromide was converted (about 5 percent) to a nontritatable species in all three solutions. The composition and results are also reported in Table II.

TABLE II

AQUEOUS PEOx-BROMINE-BROMIDE-COMPLEXES

| | % Composition | | | % Analyzed | | |
|---|---|---|---|---|---|---|
| Solution | PEOx | $Br_2$ | KBr | $Br_2$* | $Br^{\ominus}$** | Observations |
| 1 | 18 | 2 | 1.48 (1% as $Br^-$ | 1.93 | 1.05 | No $Br_2$ fumes |
| 2 | 18 | 2 | 0 | 1.6 | 0.29 | slight $Br_2$ fumes |
| 3 | 0 | 2 | 1.48 (1% as $Br^-$ | 1.92 | 0.98 | heavy $Br_2$ fumes |

* Reacted with excess KI and tritated to a starch end point with $Na_2S_2O_3$.

** Reacted with excess KI and titrated with 0.1 N $AgNO_3$ via the Volhard method. Calculated as (% total $Br_2$ via Volhard)—(% $Br_2$ via $Na_2S_2O_3$) = % as Br.

Evidence of complexing is shown by the increased capacity of water to dissolve bromine when in the presence of PEOx. PEOx-bromine complexes without bromide are not shown because the bromide spontaneously generated in solution 2.

Example 5

(Spectrophotometric Analysis of PEOx-Iodine (Triiodide) Complexes)

Triiodide ($I_3^-$) has two characteristic absorption bands at approximately 290 ($\Sigma_{max} = 45,000$) nm and 365 ($\Sigma_{max} = 25,000$) nm. These bands occur for uncomplexed triiodide and also for complexed triiodide. Several articles exist in the literature which describe typical absorption spectra for both triiodide and iodine complexes. See "Reaction of Polyamine polymer with Molecular Iodine via Charge-Transfer Complex Formation", *J. Polymer Science, 12,* 167—181, (1074) by Tomono et al. for example. Polymer-iodine complexes absorb at higher wavelengths than polymer-triiodide complexes, the latter typically in the 400—450 nm region.

A PEOx/$I_3^{\ominus}K^{\oplus}$ complex (51/49 weight ratio) was prepared in water by mixing an aqueous PEOx solution (containing 25 percent PEOx) with an aqueous $KI_3$ solution (containing 25 percent $KI_3$). The complex was then diluted to provide an iodine ($I_2$) concentration of 0.019M. This solution was analyzed spectrophotometrically and was found to exhibit the characteristic triiodide bands at 365 nm and 290 nm with respective molar extinction coefficients ($\Sigma$) of 27,000 and 32,000.

Example 6

Aqueous polymer:iodine:iodide complexes were prepared by mixing aqueous polymer solutions with the appropriate amount of Lugol's solution (5 g $I_2$, 10 g KI, 100 g $H_2O$) to obtain the desired ratios of polymer:$I_2$. The polymer plus $I_2$ concentration was 10 percent for each of the formulations although the actual percent solids (including the KI) varied from 11 percent for the 95:5 polymer:$I_2$ ratio to 17.1 percent for the 65:35 ratio. The actual percent solids was the same between directly compared PVP and PEOx samples.

TABLE III

BROOKFIELD VISCOSITY
(mPa.s at 25°C)

| Polymer:$I_2$ Ratio | PEOx | PVP |
|---|---|---|
| 95:5 | 11 | 5 |
| 85:15 | 9 | 6 |
| 75:25 | 9 | 1150 |
| 65:35 | 16 | Semi-solid (Non measurable) |

Example 7

PEOx and PVP complexes were prepared in water at a 65:35 polymer:iodine ratio and 20 percent solids as polymer plus iodine. The compositions also contained KI in an Iodine:KI ratio of 1:0.7 for a total composition of 13 percent Polymer, 7 percent Iodine, 4.9 percent KI and 75 percent $H_2O$. The compositions were prepared by adding aqueous solutions of iodine and KI to stirred aqueous polymer solutions. The compositions were then allowed to mix on a mechanical shaker for several hours. The PEOx composition was a compatible, highly fluid dark red-brown solution. The PVP composition was a multiphase, noncompatible system containing a tarlike precipitate that resisted redissolving at more dilute concentrations in $H_2O$.

Example 8

PEOx:triodide and PVP:triodide complexes having high iodine loadings were prepared in aqueous solutions. Iodine loadings of 10.7 and 16.0 percent using PEOx resulted in homogenous solutions having viscosities of 11,600 and 67,500 mPa.s, respectively. Attempts to obtain 10.5 percent $I_2$ loading using PVP resulted in a coagulated multiphase system containing portions of uncomplexed $I_2$. Results are reported in Table IV.

TABLE IV

| Polymer Content | $I_2$ Percent | Polymers:$I_2$ | $I_2$:I- (ratio) | Solids Percent | Viscosity (mPa.s, 22°C) | Comments |
|---|---|---|---|---|---|---|
| 18.1 percent PEOx | 10.7 | 63:37 | 1:0.5 | 35.7 | 11,600 | |
| 27.2 percent PEOx | 16.0 | 63:37 | 1:0.5 | 53.7 | 67,500 | |
| 30.0 percent PVP | 10.5 | 75:25 | 1:0.5 | 47.0 | Non-measurable | Multiphase contained non-complexed iodine. |

Example 9

Using an 85:15 PEOx:$I_3^-K^+$ complex, a stock solution was prepared (in soft water) containing 1000 ppm iodine (active ingredient). The PEOx had a weight average molecular weight of approximately 100,000. 0.1 ml of *E. coli.* suspension was added to 9.9 ml of a test solution containing 10 ppm iodine. After 30 seconds, the samples were neutralized with thiosulfate solution, poured into Tryptone Glucose Extract Agar plates and allowed to solidify. Control cultures (American Type Culture Collection (ATCC) #11229) were also prepared which contained 9.75× $10^4$ cells/ml. The seeded plates were then incubated at 37°C for 16 to 20 hours. Subsequent examination of the plates indicated that those treated with the polymer:triiodide complex (10 ppm $I_2$ level) exhibited no growth.

Example 10

The procedure of Example 9 was repeated except that the test solution contained 200 ppm iodine and hard water was substituted for soft water. Subsequent examination of the plates indicated that those treated with the polymer:triiodide complex exhibited no growth.

Example 11

Triiodide complexes were prepared as in Example 1 where the complexing agents were dodecylbenzene/sulfonyl chloride initiated ethyloxazoline polymers containing 5, 7 and 10 ethyloxazoline (mer) units per initiating unit. The $I_2$:iodide weight ratio was 1:0.5 (present as KI). The complexes were determined to have DC values of 100, 116 and 161 for the 5 mer, 7 mer and 10 mer, respectively.

Both the PEOx and PVP polymers used in Examples 6—8 are of the same molecular weight as their respective counterparts in Examples 1—4.

## Claims

1. A water soluble complex comprising:

(a) a polymer comprising a plurality of ring-opened units of a 2-oxazoline or 2-oxazine monomer corresponding to the formula

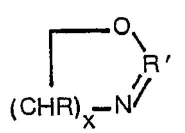

where R is hydrogen or $C_1$—$C_3$ alkyl, R′ is alkyl having 1 to 4 carbon atoms and x is 1 or 2, or the corresponding partially deacylated polymer, and

(b) $IBrCl^-$ or a polyhalide anion of the formula $(XY_{2n})^-$ where X and Y are individually chloride, bromide or iodide, but not both chloride, and n is 1, 2 or 3, and

(c) at least one independently supplied cation of hydrogen, or an alkali or alkaline earth metal.

2. The complex of Claim 1 wherein R is hydrogen and x is 1.

3. The complex of Claim 1 wherein (a) is poly(2-ethyl-2-oxazoline).

4. The complex of any one of Claims 1 to 3 wherein (b) is $I_3^-$, $IBr_2^-$, or $IBrCl^-$.

5. The complex of any one of Claims 1 to 4 wherein (a) has a weight average molecular weight between 20,000 and 500,000.

6. A process for making the water soluble complex of claim 1 characterized by contacting an aqueous solution of the polymer with I an alkali or alkaline earth metal chloride, bromide or iodide or hyrogen chloride, bromide or iodide, and II bromine or iodine.

7. The process of Claim 6 characterized in that bromine or iodine are added to the reaction mixture in the form of an aqueous solution with an alkali metal chloride, bromide or iodide or hydrogen chloride, bromide or iodide.

8. The process of Claim 6 characterized in that the chlorine, bromine or iodine are blended with a dried mixture of the polymer plus alkali metal chloride, bromine or iodide or hydrogen chloride, bromide or iodide.

9. The process of any one of Claims 6 to 8 characterized in that it is carried out at ambient temperature.

10. Complexes of (a) poly-2-oxazolines or poly-2-oxazines and (b) $IBrCl^-$ or a polyhalide anion of the formula $(xy_{2n})^-$ where x and y are individually chloride, bromide or iodide, but not both chloride, and n is 1, 2 or 3 whenever prepared by the process of any one of Claims 6 to 9.

11. Sanitizing agent characterized in that it comprises at least a complex as defined in any one of claims 1 to 5 and 10.

**Patentansprüche**

1. Ein wasserlöslicher Komplex, enthaltend:
(a) ein Polymer, bestehend aus einer Vielzahl von ringgeöffneten Einheiten eines 2-Oxazolin oder 2-Oxazin-monomer entsprechend der Formel:

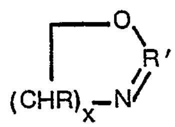

in der R Wasserstoff oder $C_1$—$C_3$ Alkyl, R' ein Alkyl mit 1—4 Kohlenstoffatomen, und $x = 1$ oder 2, oder das entsprechende deacylierte Polymer ist, und
(b) $JBrCl^-$ oder ein Polyhalogenanion der Formel $(XY_{2n})^-$ in der X und Y einzeln Chlorid, Bromid oder Jodid aber nicht beide Chloride sind, $n = 1$, 2 oder 3 ist, und
(c) wenigstens ein unabhängig geliefertes Kation des Wasserstoffs, oder eines Alkali- oder eines Erdalkalimetalls ist.

2. Der Komplex aus Anspruch 1, in dem R Wasserstoff und $x = 1$ ist.

3. Der Komplex aus Anspruch 1, in dem (a) Poly(2-ethyl-2-oxazolin) ist.

4. Der Komplex eines beliebigen der Ansprüche 1 bis 3, in dem (b) $J_3^-$, $JBr_2^-$ oder $JBrCl^-$ ist.

5. Der Komplex eines beliebigen der Ansprüche 1 bis 4, in dem
(a) ein durchschnittliches Molekulargewicht zwischen 20.000 und 500.000 hat.

6. Ein Verfahren zur Herstellung des wasserlöslichen Komplexes aus Anspruch 1, dadurch gekennzeichnet, daß eine wässrige Lösung des Polymers mit (I) einem Alkali- oder Erdalkalimetallchlorid, -bromid, oder -jodid oder Chlorwasserstoff, Bromwasserstoff oder Jodwasserstoff, und (II) Brom oder Jod in Berührung gebracht wird.

7. Verfahren aus Anspruch 6, dadurch gekennzeichnet, daß Brom oder Jod zum Reaktionsgemisch in der Form einer wäßrigen Lösung mit einem Alkalimetallchlorid-, -bromid oder -jodid bzw. Chlorwasserstoff, Bromwasserstoff oder Jodwasserstoff zugesetzt wird.

8. Verfahren nach Anspruch 6, dadurch gekennzeichnet, daß das Chlor, Brom bzw. Jod mit einem Trockengemisch des Polymers plus Alkalimetallchlorid, -bromid oder -jodid bzw. Chlorwasserstoff, Bromwasserstoff oder Jodwasserstoff vermischt wird.

9. Verfahren nach einem beliebigen der Ansprüche 6 bis 8, dadurch gekennzeichnet, daß es bei Umgebungstemperatur ausgeführt wird.

10. Komplexe aus (a) Poly-2-oxazolinen oder Poly-2-oxazinen und (b) $JBrCl^-$ oder einem Polyhalogenanion der Formel $(xy_{2n})^-$, in der x und y einzeln Chlorid, Bromid oder Jodid aber nicht beide Chlorid sind, und $n = 1$, 2 oder 3 ist, wenn sie mit einem Verfahren nach einem beliebigen der Ansprüche 6 bis 9 hergestellt werden.

11. Desinfektionsmittel, dadurch gekennzeichnet, daß es wenigstens einen Komplex der Definition nach einem beliebigen der Ansprüche 1 bis 5 und 10 enthält.

**Revendications**

1. Complexe hydrosoluble comprenant:
(a) un polymère comprenant plusieurs unités de monomère 2-oxazoline ou 2-oxazine à cycle ouvert correspondant à la formule:

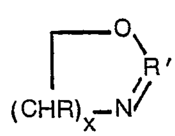

dans laquelle R est l'hydrogène ou un alkyle en $C_1$—$C_3$, R' est un alkyle ayant 1 à 4 atomes de carbone et x est égal à 1 ou 2, ou le polymère partiellement désacylé correspondant, et
(b) $IBrCl^-$ ou un anion polyhalogénure de formule $(XY_{2n})^-$ dans laquelle X et Y sont individuellement le chlorure, le bromure ou l'iodure, mais pas tous deux le chlorure, et n est égal à 1, 2 ou 3, et
(c) au moins un cation d'hydrogène, fourni indépendamment ou un métal alcalin ou alcalino-terreux.

2. Complexe selon la revendication 1, où R est l'hydrogène et x est égal à 1.

3. Complexe selon la revendication 1, où (a) est la poly(2-éthyl-2-oxazoline).

4. Complexe selon l'une quelconque des revendications 1 à 3, ou (b) est $I_3^-$, $IBr_2^-$, ou $IBrCl^-$.

5. Complexe selon l'une quelconque des revendications 1 à 4, où (a) possède un poids moléculaire moyen compris entre 20 000 et 500 000.

6. Procédé de fabrication du complexe hydrosoluble selon la revendication 1, caractérisé en ce qu'il consiste mettre en contact une solution aqueuse du polymère avec I un chlorure, bromure ou

iodure de métal alcalin ou alcalinoterreux ou du chlorure, bromure ou iodure d'hydrogène et II du brome ou de l'iode.

7. Procédé selon la revendication 6, caractérisé en ce que l'on ajoute du brome ou de l'iode au mélange réactionnel sous la forme d'une solution aqueuse avec un chlorure, bromure ou iodure de métal alcalin ou un chlorure, bromure ou iodure d'hydrogène.

8. Procédé selon la revendication 6, caractérisé en ce que l'on mélange le chlore, le brome ou l'iode avec un mélange sec du polymère plus du chlorure, bromure ou iodure de métal alcalin ou du chlorure, bromure ou iodure d'hydrogène.

9. Procédé selon l'une quelconque des revendications 6 à 8, caractérisé en ce qu'il est réalisé à la température ambiante.

10. Complexes de (a) poly-2-oxazolines ou poly-2-oxazines et de (b) $IBrCl^-$ ou d'un anion polyhalogénure de formule $(XY_{2n})^-$ dans laquelle X et Y sont individuellement le chlorure, le bromure ou l'iodure, mais pas tous deux le chlorure, et n est égal à 1, 2 ou 3, préparés par un procédé selon l'une quelconque des revendications 6 à 9.

11. Désinfectant caractérisé en ce qu'il comprend au moins un complexe selon la définition de l'une quelconque des revendications 1 à 5 et 10.